(19) Europäisches Patentamt
European Patent Office
Office européen des brevets

(11) EP 1 306 074 A1

(12) # DEMANDE DE BREVET EUROPEEN

(43) Date de publication:
**02.05.2003 Bulletin 2003/18**

(51) Int Cl.7: **A61K 7/02**

(21) Numéro de dépôt: **02292479.9**

(22) Date de dépôt: **08.10.2002**

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **25.10.2001 FR 0113818**

(71) Demandeur: **L'OREAL**
**75008 Paris (FR)**

(72) Inventeurs:
- **Yousfi, Naime**
**75013 Paris (FR)**
- **Piot, Bertrand**
**75009 Paris (FR)**
- **Senee, Jérôme**
**91510 Lardy (FR)**

(74) Mandataire: **Rasson, Catherine**
**L'OREAL-DPI**
**6 rue Bertrand Sincholle**
**92585 Clichy Cedex (FR)**

(54) **Composition topique solide**

(57) La présente demande concerne une composition solide comprenant une phase grasse comportant au moins un éther de polyol, au moins une huile et au moins une cire ayant une polarité inverse de celle de l'huile.

L'éther de polyol peut être notamment un éther de pentaérythritol ou un éther de sucre.

Cette composition peut constituer notamment un stick et elle peut être utilisée en tant que produit de soin et/ou de maquillage des matières kératiniques, notamment de la peau, des lèvres et/ou des phanères.

EP 1 306 074 A1

Printed by Jouve, 75001 PARIS (FR)

## Description

La présente invention a trait à une composition solide comprenant une phase grasse comportant au moins un éther de polyol, au moins une huile et au moins une cire. Cette composition est susceptible de constituer un stick et d'être utilisée comme composition de maquillage et/ou de soin des matières kératiniques telles la peau, les lèvres et les phanères.

Les compositions sous forme solide et notamment sous forme de stick contiennent généralement des cires utilisées pour solidifier la composition et obtenir un stick sous forme de bâton. Néanmoins, la présence d'une quantité trop importante de cires donnent un stick ayant des qualités cosmétiques insuffisantes, et notamment présentant des difficultés d'étalement et donnant de faibles dépôts sur la peau ou les lèvres.

Pour améliorer les propriétés cosmétiques des sticks contenant des cires, une solution consiste à ajouter des huiles afin d'obtenir un bon étalement et un meilleur dépôt de la composition. Cependant, les compositions comportant cire et huile fondent généralement à des températures inférieures ou égales à 37°C, ce qui a pour inconvénient de donner des sticks fragiles, ayant en outre un aspect huileux et exsudant fortement.

On a essayé de remédier à ces inconvénients en ajoutant des charges qui ont l'avantage d'aider à la tenue du stick lors d'élévation de température et d'améliorer les qualités cosmétiques du stick. Toutefois, l'ajout de charges peut donner des sticks cassants et durs, voire même parfois rêches.

Il subsiste donc le besoin d'une composition solide pouvant se présenter sous la forme d'un stick, stable en température, notamment stable aux étuves, en particulier ne présentant pas d'exsudation lors d'élévation de la température, tout en fondant sur la matière kératinique où elle est appliquée, par exemple peau ou lèvres, et ce même en absence de charges.

La demanderesse a découvert de manière surprenante, que l'ajout d'éther de polyol dans un mélange d'huile et de cire permettait d'obtenir un stick qui ne fonde pas à des températures inférieures ou égales à 37°C, tout en ayant un bon étalement et un bon dépôt sur les lèvres ou la peau. La composition obtenue présente l'avantage de posséder en outre de bonnes propriétés cosmétiques, et en particulier de ne pas procurer de sensation de sécheresse lorsqu'elle est appliquée sur la peau. La composition selon l'invention peut en outre être avantageusement utilisée pour traiter les symptômes liés au dessèchement de la peau et/ou des lèvres.

La présente invention a donc pour objet une composition solide comprenant dans un milieu physiologiquement acceptable, au moins une phase grasse comportant au moins un éther de polyol, au moins une huile et au moins une cire ayant une polarité inverse de celle de l'huile.

Utiliser une cire ayant une « polarité inverse de celle de l'huile » signifie ici que lorsque la composition comprend une huile polaire, elle doit contenir au moins une cire apolaire, et que, lorsque la composition comprend une huile apolaire, elle doit contenir au moins une cire polaire. Quand la composition comprend à la fois une huile polaire et une huile apolaire, elle doit comprendre au moins une cire apolaire.

La demanderesse a trouvé que les bonnes propriétés de stabilité et de délitement (étalement et dépôt sur les lèvres ou la peau) de la composition de l'invention sont liées à une certaine dureté de la composition, dureté qui est liée à la force de cisaillement. Pour obtenir de propriétés adéquates, la dureté et donc la force de cisaillement de la composition selon l'invention doivent être de préférence telles que la composition soit autoportée, c'est-à-dire qu'elle se supporte elle-même en restant sous sa forme solide (par exemple bâton) et ne s'affaisse pas sous son poids comme le font les crèmes ou les liquides, et qu'elle puisse se déliter aisément pour former un dépôt satisfaisant sur la peau et les lèvres. La dureté des sticks obtenus est mesurée à 20°C au moyen d'un dynamomètre DFGHS 2 de la société INDELCO-CHATILLON se déplaçant à une vitesse de 100 mm/minute. Elle est exprimée comme la force de cisaillement (exprimée en grammes) nécessaire pour couper un stick de 12,7 mm de diamètre dans ces conditions. Dans la présente demande, la force de cisaillement de la composition va de préférence de 100 à 300 g, mieux de 120 à 250 g, et encore mieux de 150 à 220 g.

Ainsi, la présente invention a aussi pour objet une composition solide comprenant dans un milieu physiologiquement acceptable, au moins une phase grasse comportant au moins un éther de polyol, au moins une cire et au moins une huile, la dite composition ayant une force de cisaillement allant de 100 à 300 g.

D'une manière générale, la composition selon l'invention constitue une composition solide et elle peut se présenter sous forme d'un produit coulé ou en coupelle ou sous forme d'un stick.

La composition solide selon l'invention étant destinée à une application topique, notamment sur la peau, les lèvres ou les phanères, elle comprend un milieu physiologiquement acceptable. On entend ici par « milieu physiologiquement acceptable », un milieu non toxique et susceptible d'être appliqué sur la peau (y compris l'intérieur des paupières), les lèvres, les ongles ou les cheveux d'êtres humains. La composition de l'invention peut constituer notamment une composition cosmétique ou dermatologique.

Par ailleurs, on entend par « composition solide » au sens de la présente invention, toute composition ne s'écoulant pas sous son propre poids, présentant une dureté telle que définie ci-dessus (force de cisaillement de 100 à 300 g).

La composition de l'invention comprend de préférence une seule phase grasse, et cette phase grasse est de préférence une phase continue.

Les compositions de l'invention peuvent être des compositions anhydres, c'est-à-dire exemptes

d'eau ou de composés hydrophiles, mais elles peuvent aussi ne pas être anhydres et comprendre alors jusqu'à 10 % en poids de phase hydrophile par rapport au poids total de la composition, de préférence 1 à 5 % en poids de phase hydrophile et mieux de 1 à 2 % de phase hydrophile par rapport au poids total de la composition, la phase hydrophile étant constituée d'eau seule, ou d'eau et d'additifs hydrophiles et hydrosolubles tels que polyols, gélifiants et/ou actifs. Si cette phase hydrophile est présente, elle est de préférence dispersée dans la phase grasse qui forme une phase continue.

Selon un mode particulier de réalisation de l'invention, la composition est anhydre, c'est-à-dire qu'elle ne comporte que la phase grasse, ou quasiment anhydre, c'est-à-dire qu'elle comporte moins de 5 % en poids d'eau et/ou additifs hydrophiles ou hydrosolubles.

Dans la composition selon l'invention, l'éther de polyol peut être choisi notamment parmi les éthers de pentaérythritol et de polyalkylène glycol, les éthers d'alcool gras et de sucre, et leurs mélanges.

Les éthers de pentaérythritol et de polyalkylène glycol peuvent comporter notamment de 1 à 450 motifs oxyalkylénés, de préférence de 1 à 200 motifs oxyalkylénés, mieux 1 à 100 motifs oxyalkylénés et encore mieux de 1 à 50 motifs oxyalkylénés. Ils peuvent être en particulier choisis parmi les éthers de pentaérythritol et de polyéthylène glycol comportant de 1 à 450 motifs oxyéthylénés, de préférence de 1 à 200 motifs oxyéthylénés, mieux 1 à 100 motifs oxyéthylénés et encore mieux de 1 à 50 motifs oxyéthylénés ; les éthers de pentaérythritol et de polypropylène glycol comportant de 1 à 450 motifs oxypropylénés, de préférence de 1 à 200 motifs oxypropylénés, mieux 1 à 100 motifs oxypropylénés et encore mieux de 1 à 50 motifs oxypropylénés ; et leurs mélanges. Selon un mode préféré de réalisation de l'invention, on utilise l'éther pentaérythritol et de polyéthylène glycol comportant 5 motifs oxyéthylénés (5 OE) (nom CTFA : PEG-5 Pentaerythrityl Ether), l'éther de pentaérythritol et de polypropylène glycol comportant 5 motifs oxypropylénés (5 OP) (nom CTFA : PPG-5 Pentaerythrityl Ether), et leurs mélanges et plus spécialement le mélange PEG-5 Pentaerythrityl Ether, PPG-5 Pentaerythrityl Ether et huile de soja, commercialisé sous la dénomination « Lanolide » par la société Vevy, mélange où les constituants se trouvent dans un rapport en poids 46/46/8 : 46 % de PEG-5 Pentaerythrityl Ether, 46 % de PPG-5 Pentaerythrityl Ether et 8 % d'huile de soja.

Les éthers d'alcool gras et de sucre peuvent être choisis dans le groupe comprenant les éthers ou mélanges d'éthers d'alcool gras en $C_8$-$C_{22}$ et de glucose, de maltose, de sucrose ou de fructose ; les éthers ou mélanges d'éthers d'alcool gras en $C_{14}$-$C_{22}$ et de méthylglucose ; et leurs mélanges.

Les alcools gras en $C_8$-$C_{22}$ ou en $C_{14}$-$C_{22}$ formant le motif gras des éthers de sucre comportent une chaîne alkyle linéaire saturée ou non saturée, comportant respectivement de 8 à 22 ou de 14 à 22 atomes de carbone. Le motif gras des éthers provenant de l'alcool gras peut être notamment choisi parmi les motifs décyle, cétyle, béhényle, arachidyle, stéaryle, palmityle, myristyle, lauryle, capryle, hexadécanyle, et leurs mélanges tels que cétéaryle (mélange de cétyle et de stéaryle).

A titre d'exemples d'éthers d'alcool gras et de sucre, on peut citer en particulier les alkylpolyglucosides (APG) tels que le décylglucoside et le laurylglucoside commercialisés par exemple par la société Henkel sous les dénominations respectives Plantaren 2000 et Plantaren 1200, le cétéarylglucoside éventuellement en mélange avec l'alcool cétostéarylique, le dit mélange étant commercialisé par exemple sous la dénomination Montanov 68 par la société Seppic (mélange où les constituants se trouvent dans un rapport 12/46/42 : 12 % de Cétéarylglucoside, 46 % d'alcool cétylique et 42 % d'alcool stéarylique). Comme cétéarylglucoside, on peut citer aussi les produits commercialisés sous la dénomination Tegocare CG90 par la société Goldschmidt et sous la dénomination Emulgade KE3302 par la société Henkel. Comme APG, on peut citer aussi l'arachidyl glucoside, par exemple sous la forme du mélange d'alcools arachidique et béhénique et d'arachidylglucoside, commercialisé sous la dénomination Montanov 202 par la société Seppic. On peut citer aussi les alkylpolyglucosides à chaîne ramifiée ou insaturée, tels que l'isostéaryl-glucoside éventuellement en mélange avec l'alcool isostéarylique, commercialisé par exemple sous la dénomination Montanov WO18 par la société Seppic, et l'oléylglucoside éventuellement en mélange avec l'alcool oléylique, commercialisé par la société Seppic. On peut utiliser aussi un mélange de ces alkylpolyglucosides. Selon un mode particulier de réalisation de l'invention, le mélange de l'alkylpolyglucoside tel que défini ci-dessus avec l'alcool gras correspondant peut être sous forme d'une composition autoémulsionnante, comme décrit par exemple dans le document WO-A-92/06778.

La quantité d'éther(s) de polyol peut varier dans une large mesure, et elle peut aller par exemple de 0,5 à 40 % en poids, de préférence de 1 à 30 % en poids et mieux de 5 à 25 % en poids par rapport au poids total de la composition.

La phase grasse de la composition selon l'invention comprend au moins une huile. Par "huile", on entend tout milieu non aqueux liquide à température ambiante (25°C) et pression atmosphérique (760 mm de Hg), physiologiquement acceptable.

La quantité d'huile(s) peut aller par exemple de 20 à 80 % en poids et de préférence de 30 à 70 % en poids par rapport au poids total de la composition.

Ces huiles peuvent être des huiles hydrocarbonées et/ou siliconées et/ou fluorées. Elles peuvent être d'origine animale, végétale, minérale ou synthétique. On entend par « huile hydrocarbonée », toute huile comportant majoritairement des atomes de carbone et d'hydrogène, et éventuellement des groupements ester, éther, fluoré, acide carboxylique et/ou alcool. En outre, les huiles utilisées peuvent être volatiles et/ou non volatiles. Par huile volatile, on entend une huile susceptible de s'évaporer à température ambiante d'un support sur lequel elle a été appliquée, autrement dit une huile ayant une tension de vapeur mesurable à 25°C et 1 atmosphère, par exemple supérieure à 0 Pa, en particulier allant de $10^{-3}$ à 300 mm de Hg (0,13 Pa à 40.000 Pa). On peut notamment citer comme huiles volatiles, les huiles siliconées volatiles, telles que les silicones volatiles cycliques ou linéaires. On peut également citer les huiles volatiles hydrocarbonées telles que les isoparaffines, et les huiles fluorées volatiles.

Parmi les huiles pouvant être utilisées dans la composition de l'invention, certaines sont polaires et d'autres sont apolaires (c'est-à-dire non polaires).

Les huiles polaires comportent dans leur structure chimique au moins un groupement polaire non ionique, et de préférence au moins deux groupements polaires non ioniques ou ioniques tels que les groupements suivants :

- COOH ;
- OH mono ou disubstitué (primaire ou secondaire) ;
- $PO_4$ ;
- NHR ; $NR_1R_2$, $R_1$ et $R_2$ formant éventuellement un cycle et représentant un radical alkyle ou alcoxy linéaire ou ramifié en $C_1$ à $C_{20}$, ou

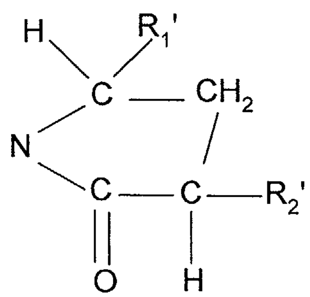

où $R_1$' et $R_2$' peuvent représenter l'hydrogène ou une chaîne alkyle ou alkoxy linéaire ou ramifiée en $C_1$ à $C_{20}$.

La polarité peut être décrite par le paramètre de solubilité de Hansen $\delta_a$. En effet, ce paramètre caractérise, pour un constituant donné, l'énergie correspondant aux interactions polaires ($\delta_p$) et de types liaisons hydrogène ($\delta_h$) existant entre les molécules de ce constituant.

$$\delta_a = \sqrt{\delta_p^2 + \delta_h^2}$$

Les huiles apolaires ont une valeur de $\delta_a$ égale à 0. En particulier, les huiles apolaires selon l'invention peuvent être en particulier choisies parmi :

- les huiles de silicone, telles que les polydiméthylsiloxanes (PDMS) volatils ou non, linéaires ou cycliques, liquides à température ambiante ; les silicones phénylées comme les phényltriméthicones, les phényldiméthicones, les phényltriméthylsiloxydiphénylsiloxanes, des diphényldiméthicones, les diphénylméthyldiphényltrisiloxanes, les 2-phényléthyl triméthylsiloxysilicates ;
- les hydrocarbures, linéaires ou ramifiés, d'origine synthétique ou minérale, comme les huiles de paraffine, volatiles ou non volatiles, et leurs dérivés ; l'huile de vaseline ; la lanoline liquide ; les polydécènes ; le polyisobutène hydrogéné tel que l'huile de Parléam® ; le squalane ; l'isoparaffine hydrogénée ; l'isohexadecane ; l'isododecane ;
- et leurs mélanges.

Les huiles polaires ont une valeur de $\delta_a$ différente de 0, c'est-à-dire supérieure à 0. En particulier, les huiles polaires utilisées dans la composition de l'invention peuvent être choisies parmi :

- les huiles d'origine végétale, huiles hydrocarbonées à forte teneur en triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées. Comme huiles d'origine végétale, on peut citer notamment les huiles de jojoba, de germes de blé, de maïs, de tournesol, de beurre de karité, de ricin, d'amandes douces, de macadamia, d'abricot, de soja, de coton, de luzerne, de pavot, de potimarron, de sésame, de courge, de colza, d'avocat, de noisette, de pépins de raisin ou de cassis, d'onagre, de millet, d'orge, de quinoa, d'olive, de seigle, de carthame, de bancoulier, de passiflore, de rosier muscat, de coriandre ; ou encore les triglycérides des acides caprylique/caprique comme ceux vendus par la société Stearineries Dubois ou ceux vendus sous les dénominations Miglyol 810, 812 et 818 par la société Dynamit Nobel ;
- les huiles de synthèse ou esters de synthèse de formule $R_5COOR_6$ dans laquelle $R_5$ représente le reste d'un acide gras linéaire ou ramifié comportant de 1 à 40 atomes de carbone et $R_6$ représente une chaîne hydrocarbonée notamment ramifiée contenant de 1 à 40 atomes de carbone à condition que $R_5 + R_6$ soit $\geq$ 10, comme par exemple l'huile de Purcellin (octanoate de cétostéaryle), l'isononanoate d'isononyle, le benzoate d'alcool en $C_{12}$ à $C_{15}$, le myristate d'isopropyle, le palmitate d'éthyl 2-hexyle, l'isostéarate d'isostéaryle, l'isostéarate d'isopropyle, des octanoates, décanoates ou ricinoléates d'alcools ou de polyalcools ; les esters hydroxylés comme le lactate d'isostéaryle, le malate de diisostéaryle ; le C12-C15 alkylbenzoate ; et les esters du pentaérythritol ;
- les éthers de synthèse ayant de 10 à 40 atomes de carbone ;
- les alcools gras en $C_8$ à $C_{26}$, comme l'alcool oléi-

que, l'alcool isostéarylique, et l'octyldodécanol ;

- leurs mélanges.

La phase grasse de la composition selon l'invention comprend au moins une cire. Une cire, au sens de la présente invention, est un composé gras lipophile, solide à température ambiante (25°C), à changement d'état solide/liquide réversible, ayant une température de fusion supérieure à 40°C et pouvant aller jusqu'à 200°C, et présentant à l'état solide une organisation cristalline anisotrope.

On peut utiliser tout type de cire. Les cires peuvent être choisies parmi les cires d'origine naturelle, notamment d'origines végétale ou animale, parmi les cires d'origine minérale, parmi les cires d'origine synthétique, et leurs mélanges. Comme cires pouvant être utilisées dans la composition de l'invention, on peut citer par exemple la cire d'abeilles, la cire de Montan, la cire de Carnauba, la cire de Candellila, la cire de Chine, la cire de lin, la cire de sapin, la cire de coton, la cire d'Ouricoury, la cire de lignite, la cire de son de riz, la cire de canne à sucre, la cire du Japon, la cire de fibres de liège, les cires de paraffine, les cires microcristallines, la cire de lanoline, les ozokérites, les huiles hydrogénées ayant une température de fusion supérieure à 40°C (environ), comme l'huile de jojoba hydrogénée, les cires de polyéthylène qui sont issues de la polymérisation de l'éthylène, les cires obtenues par synthèse de Fischer-Tropsch, les esters d'acides gras et les glycérides concrets (c'est-à-dire solides) à 40°C, les cires de silicone comme les alkyle, alcoxy et/ou esters de poly(di)méthylsiloxane solides à 40°C ; et leurs mélanges.

Comme indiqué ci-dessus, quand la composition comprend une huile polaire, elle doit contenir au moins une cire apolaire. Quand la composition comprend une huile apolaire, elle doit contenir au moins une cire polaire. Quand la composition comprend une huile polaire et une huile apolaire, elle doit comprendre au moins une cire apolaire.

Comme indiqué ci-dessus pour les huiles, la polarité peut être décrite par le paramètre de solubilité de Hansen $\delta_a$ selon l'équation indiquée ci-dessus.

Les cires dites apolaires ont une valeur de $\delta_a$ égale à 0. Ce sont notamment les cires hydrocarbonées comportant essentiellement des atomes de carbone et d'hydrogène, ou des cires de silicone. Comme cires hydrocarbonées, on peut citer en particulier les cires microcristallines, l'ozokerite, les cires de paraffine, les cires de polyéthylène (non modifiées).

Les cires dites polaires sont des cires comportant des groupements polaires tels qu'indiqués ci-dessus pour les huiles, et elles ont une valeur de $\delta_a$ supérieure à 0. Ce sont notamment les cires d'origine animale, les cires d'origine végétale, les cires d'origine synthétique comportant des groupements polaires et les cires de silicone comportant des groupements polaires. On peut citer par exemple la cire de Montan, la cire de Carnauba, la cire de Candellila, la cire de Chine, la cire de lin, la cire de sapin, la cire de coton, la cire d'Ouricoury, la cire de lignite, la cire de son de riz, la cire de canne à sucre, la cire du Japon, la cire de fibres de liège, les cires d'abeille polyglycérolées, les huiles hydrogénées, les esters d'acides gras et les glycérides concrets à 40°C, les cires de silicone comportant un ou plusieurs groupes ester.

La quantité totale de cire(s) (polaires et/ou apolaires) peut aller par exemple de 5 à 40 % en poids et de préférence de 10 à 30 % en poids par rapport au poids total de la composition.

Selon un mode particulier de réalisation de l'invention, la composition comprend au moins une cire microcristalline et au moins une huile polaire, et notamment une huile choisie parmi les huiles d'origine végétale, les alcools gras, et leurs mélanges, et plus particulièrement l'octyldodécanol, l'huile de beurre de karité, l'huile de ricin, l'huile d'avocat et leurs mélanges. Une telle composition peut comprendre en outre une ou plusieurs huiles apolaires, notamment celles choisies parmi les hydrocarbures linéaires ou ramifiés, tels que les polydécènes et l'huile de Parléam par exemple, et/ou une ou plusieurs autres cires, polaires ou apolaires.

Comme indiqué ci-dessus, la composition de l'invention peut comprendre de 0 à 10 % en poids d'une phase hydrophile, par rapport au poids total de la composition, et mieux de 1 à 5 % en poids, pouvant comprendre de l'eau et/ou des additifs hydrophiles ou hydrosolubles (actifs et/ou gélifiants par exemple). Elle peut notamment comprendre des hydratants tels que la glycérine. Les constituants hydrophiles éventuellement présents sont de préférence dispersés dans la phase grasse constituée des huiles et cires.

La composition selon l'invention peut comprendre en outre une phase particulaire. La quantité de phase particulaire peut aller par exemple de 0 à 30 % en poids, de préférence de 0 à 20 % en poids par rapport au poids total de la composition. Quand une phase particulaire est présente, sa quantité est généralement d'au moins 0,05 % en poids par rapport au poids total de la composition. La quantité de phase particulaire peut aller par exemple de 0,05 à 30 % en poids et de préférence de 1 à 20 % en poids par rapport au poids total de la composition. Cette phase particulaire peut comprendre des particules choisies parmi les pigments, les nacres, les charges, et leurs mélanges. Ces pigments, nacres, et charges sont choisis parmi ceux habituellement utilisés dans les compositions cosmétiques. Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.

Les pigments peuvent être blancs ou colorés,

minéraux et/ou organiques, de taille micrométrique ou nanométrique. On peut citer par exemple, parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.

Parmi les nacres utilisables dans la composition de l'invention, on peut citer par exemple le mica recouvert d'oxyde de titane, d'oxyde de fer, de pigment naturel ou d'oxychlorure de bismuth ainsi que le mica titane coloré.

Les charges peuvent être minérales ou de synthèse, lamellaires ou non lamellaires. Comme charges utilisables dans la composition de l'invention, on peut citer par exemple le talc, le mica, la silice, le kaolin, les poudres de Nylon, les poudres de polyéthylène, le Téflon, l'amidon modifié ou non, le micatitane, la nacre naturelle, le nitrure de bore, les microsphères telles que l'Expancel (Nobel Industrie), le Polytrap (Dow Corning) et les microbilles de résine de silicone (Tospearls de Toshiba, par exemple).

Avantageusement, la composition selon l'invention comprend au moins une matière colorante qui peut être choisie parmi les colorants lipophiles ou les colorants hydrophiles habituellement utilisés dans les compositions cosmétiques ou dermatologiques, ainsi que parmi les pigments et les nacres décrits ci-dessus ; et leurs mélanges. Cette matière colorante est généralement présente à raison de 0,01 à 40 % en poids et de préférence de 5 à 25 % en poids par rapport au poids total de la composition.

La composition selon l'invention peut comprendre en outre tout additif usuellement utilisé dans le domaine considéré, notamment le domaine cosmétique, tel que les antioxydants ; les parfums ; les huiles essentielles ; les conservateurs ; les actifs cosmétiques ; les vitamines comme la vitamine E (tocophérol) et ses dérivés (par exemple acétate), la vitamine A (rétinol) et ses dérivés (par exemple palmitate de rétinyle), la vitamine C (acide ascorbique) et ses dérivés (par exemple palmitate d'ascorbyle), les dérivés de ces vitamines étant notamment des esters dont le palmitate et l'acétate ; les acides gras essentiels ; les sphingolipides et céramides ; les composés auto-bronzants tels que la DHA (dihydroxyacétone) ; les filtres solaires comme par exemple l'octylméthoxycinnamate (Parsol MCX), la 3-benzophénone (Uvinul M40), le butylméthoxydibenzoyl-méthane (Parsol 1789) ; les tensioactifs ; les polymères ; et leurs mélanges. Ces additifs peuvent être présents dans la composition à raison de 0 à 20 % en poids par rapport au poids total de la composition.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Comme indiqué ci-dessus, la composition selon l'invention se présente sous forme solide. On entend par là qu'on n'observe aucun affaissement de la composition en dehors du récipient la comprenant, en l'absence de stimulation mécanique ou thermique (chauffage notamment).

Les procédés de fabrication des compositions selon l'invention ne diffèrent en rien des procédés classiquement utilisés en cosmétique et parfaitement connus de l'homme de l'art.

Les compositions selon l'invention peuvent notamment constituer un produit de soin et/ou un produit de maquillage des matières kératiniques, notamment de la peau, des lèvres et des phanères comme les ongles, les cils, les sourcils et les cheveux. Les produits de maquillage sont le plus souvent colorés et contiennent généralement des pigments. Sous forme de produits de maquillage, les compositions de l'invention peuvent constituer avantageusement un fond de teint, un rouge à lèvres, un fard à joues, un fard à paupières, un mascara ou un eyeliner.

Ce produit peut être sous forme d'une poudre coulée, d'un produit en coupelle (fond de teint, fard à joues, fard à paupières), ou d'un produit sous forme de bâton (rouge à lèvres ou stick de soin des lèvres). Selon un mode préféré de réalisation de l'invention, elle se présente sous forme de bâton (stick), plus particulièrement pour le soin des lèvres ou pour le maquillage des lèvres comme rouge à lèvres.

La composition de l'invention peut être utilisée pour le soin et/ou le traitement de la peau, des lèvres et/ou des phanères, par exemple pour l'hydratation des lèvres et/ou pour le traitement des lèvres gercées et/ou sèches.

Aussi, l'invention a aussi pour objet l'utilisation cosmétique d'une composition cosmétique telle que définie ci-dessus, pour le soin et/ou le traitement de la peau, des lèvres et/ou des phanères, et en particulier pour l'hydratation des lèvres.

L'invention a aussi pour objet l'utilisation cosmétique d'une composition cosmétique telle que définie ci-dessus, pour le maquillage de la peau, des lèvres et/ou des phanères.

L'invention a aussi pour objet un procédé cosmétique de traitement des lèvres gercées et/ou sèches, consistant à appliquer sur les lèvres, une composition cosmétique telle que définie ci-dessus.

L'invention est illustrée plus en détail dans les exemples suivants, dans lesquels les pourcentages sont donnés en poids, sauf mention contraire.

| **Exemple 1 : stick de soin** | |
|---|---|
| Lanolide | 15 % |
| Cire microcristalline | 15 % |
| Ozokerite | 5 % |

(suite)

| Exemple 1 : stick de soin | |
|---|---|
| Huile polaire (huile de ricin) | 35 % |
| Huile non polaire (huile de Parléam) | 27 % |
| Filtre (Parsol MCX) | 1 % |
| Vitamine E | 1 % |
| Parfum | 1 % |

Mode opératoire : la lanolide, les huiles et les actifs (filtre, vitamine E et parfum) (phase A) sont chauffées vers 85°C sous agitation. Les cires (phase B) sont chauffées vers 100°C. Après fusion complète des cires, on ajoute la phase A dans la phase B. Le mélange obtenu est ensuite coulé dans des moules et refroidi à température ambiante.

On obtient un produit coulé stable (bonne stabilité en température), ayant de bonnes propriétés cosmétiques (bon dépôt sur la peau ou les lèvres et douceur) et utilisable par exemple pour protéger les lèvres et éviter leur dessèchement.

| Exemple 2 : stick de soin | |
|---|---|
| Lanolide | 13 % |
| Cire microcristalline | 20 % |
| Ozokerite | 5 % |
| Huile polaire (huile de ricin) | 32 % |
| Huile non polaire (polydécène) | 27 % |
| Palmitate d'ascorbyle | 1 % |
| Vitamine E | 1 % |
| Parfum | 1 % |

Le mode opératoire est identique à celui de l'exemple 1.

On obtient un stick stable (bonne stabilité en température), ayant de bonnes propriétés cosmétiques (bon dépôt sur la peau ou les lèvres et douceur) et utilisable par exemple pour traiter les lèvres gercées.

| Exemple 3 : stick de soin | |
|---|---|
| Montanov 68 | 17 % |
| Cire microcristalline | 15 % |
| Cire d'abeille polyglycérolée | 5 % |
| Huile polaire (huile de ricin) | 15 % |
| Huile polaire (huile d'avocat) | 20 % |
| Huile non polaire (huile de Parléam) | 25 % |
| Palmitate d'ascorbyle | 1 % |
| Palmitate de rétinyle | 1 % |
| Parfum | 1 % |

Le mode opératoire est identique à celui de l'exemple 1.

On obtient un stick stable (bonne stabilité en température), ayant de bonnes propriétés cosmétiques (bon dépôt sur la peau ou les lèvres et douceur) et utilisable par exemple pour traiter les lèvres sèches.

| Exemple 4 : Rouge à lèvres | |
|---|---|
| Lanolide | 20 % |
| Cire microcristalline | 17 % |
| Huile polaire (octyldodécanol) | 21 % |
| Huile non polaire (huile de Parléam) | 20 % |
| Huile non polaire (polydécène) | 10 % |
| CI 77491 (colorant brun) | 11 % |
| Allantoïne | 0,5 % |
| Acétate de tocophérol | 0,5 % |

Le mode opératoire est identique à celui de l'exemple 1.

On obtient un stick coloré stable (bonne stabilité en température), ayant de bonnes propriétés cosmétiques (bon dépôt sur la peau ou les lèvres et douceur) et utilisable comme rouge à lèvres.

## Revendications

1. Composition solide comprenant dans un milieu physiologiquement acceptable, au moins une phase grasse comportant au moins un éther de polyol, au moins une huile et au moins une cire ayant une polarité inverse de celle de l'huile.

2. Composition solide comprenant dans un milieu physiologiquement acceptable, au moins une phase grasse comportant au moins un éther de polyol, au moins une cire et au moins une huile, la dite composition ayant une force de cisaillement allant de 100 à 300 g.

3. Composition selon l'une quelconque des revendications précédentes, **caractérisée en ce que** l'éther de polyol est choisi parmi les éthers de pentaérythritol et de polyalkylène glycol, les éthers d'alcool gras et de sucre, et leurs mélanges.

4. Composition selon l'une quelconque des revendications précédentes, **caractérisée en ce que** l'éther de polyol est choisi parmi les éthers de pentaérythritol et de polyéthylène glycol comportant de 1 à 450 motifs oxyéthylénés, les éthers de pentaérythritol et de polypropylène glycol comportant de 1 à 450 motifs oxypropylénés, et leurs mélanges.

5. Composition selon la revendication précédente, **caractérisée en ce que** l'éther de polyol est choisi parmi l'éther de pentaérythritol et de polyéthylène glycol comportant 5 motifs oxyéthylénés, l'éther de pentaérythritol et de polypropylène glycol comportant 5 motifs oxypropylénés, et leurs mélanges.

**6.** Composition selon la revendication précédente, **caractérisée en ce que** l'éther de polyol est sous forme d'un mélange éther de pentaérythritol et de polyéthylène glycol comportant 5 motifs oxyéthylénés / éther de pentaérythritol et de polypropylène glycol comportant 5 motifs oxypropylénés / huile de soja.

**7.** Composition selon l'une quelconque des revendications précédentes, **caractérisée en ce que** l'éther de polyol est choisi parmi les éthers ou mélanges d'éthers d'alcool gras en $C_8$-$C_{22}$ et de glucose, de maltose, de sucrose ou de fructose ; les éthers ou mélanges d'éthers d'alcool gras en $C_{14}$-$C_{22}$ et de méthylglucose ; et leurs mélanges.

**8.** Composition selon la revendication précédente, **caractérisée en ce que** l'éther de polyol est choisi parmi les alkylpolyglucosides.

**9.** Composition selon la revendication précédente, **caractérisée en ce que** l'alkylpolyglucoside est choisi parmi le décylglucoside, le laurylglucoside, le cétéarylglucoside, l'arachidyl glucoside, l'isostéarylglucoside, l'oléyl-glucoside et leurs mélanges.

**10.** Composition selon l'une quelconque des revendications précédentes, **caractérisée en ce que** la quantité d'éther(s) de polyol va de 0,5 à 40 % en poids et de préférence de 1 à 30 % en poids par rapport au poids total de la composition.

**11.** Composition selon l'une quelconque des revendications précédentes, **caractérisée en ce que** la quantité d'huile(s) va de 20 à 80 % en poids, et de préférence de 30 à 70 % en poids par rapport au poids total de la composition.

**12.** Composition selon l'une quelconque des revendications précédentes, **caractérisée en ce que** l'huile est une huile apolaire choisie parmi les huiles de silicone ; les hydrocarbures linéaires ou ramifiés ; et leurs mélanges.

**13.** Composition selon l'une quelconque des revendications précédentes, **caractérisée en ce que** l'huile est une huile polaire choisie parmi les huiles d'origine végétale ; les huiles de synthèse ou esters de synthèse de formule $R_5COOR_6$ dans laquelle $R_5$ représente le reste d'un acide gras linéaire ou ramifié comportant de 1 à 40 atomes de carbone et $R_6$ représente une chaîne hydrocarbonée notamment ramifiée contenant de 1 à 40 atomes de carbone à condition que $R_5 + R_6$ soit $\geq 10$ ; les éthers de synthèse ; les alcools gras en $C_8$ à $C_{26}$ ; et leurs mélanges.

**14.** Composition selon l'une quelconque des revendications précédentes, **caractérisée en ce que** la cire est choisie parmi les cires d'origine naturelle ; les cires d'origine minérale, les cires d'origine synthétique ; et leurs mélanges.

**15.** Composition selon la revendication précédente, **caractérisée en ce que** la cire est choisie parmi la cire d'abeilles, la cire de Montan, la cire de Carnauba, la cire de Candellila, la cire de Chine, la cire de lin, la cire de sapin, la cire de coton, la cire d'Ouricoury, la cire de lignite, la cire de son de riz, la cire de canne à sucre, la cire du Japon, la cire de fibres de liège, les cires de paraffine, les cires microcristallines, la cire de lanoline, les ozokérites, les huiles hydrogénées ayant une température de fusion supérieure à 40°C, les cires de polyéthylène, les cires obtenues par synthèse de Fischer-Tropsch, les esters d'acides gras et les glycérides concrets à 40°C, les cires de silicone, et leurs mélanges.

**16.** Composition selon l'une quelconque des revendications précédentes, **caractérisée en ce que** la quantité de cire(s) va de 5 à 40 % en poids et de préférence de 10 à 30 % en poids par rapport au poids total de la composition.

**17.** Composition selon l'une quelconque des revendications précédentes, **caractérisée en ce qu'**elle comprend au moins une cire microcristalline et au moins une huile polaire choisie parmi les huiles d'origine végétale, les alcools gras, et leurs mélanges.

**18.** Composition selon l'une quelconque des revendications précédentes, **caractérisée en ce qu'**elle comprend de 0 à 10 % en poids d'une phase hydrophile.

**19.** Composition selon la revendication précédente, **caractérisée en ce que** la phase hydrophile comprend de l'eau et/ou des additifs hydrophiles ou hydrosolubles.

**20.** Composition selon l'une quelconque des revendications précédentes, **caractérisée en ce qu'**elle comprend une phase particulaire.

**21.** Composition selon la revendication précédente, **caractérisée en ce que** la phase particulaire comprend des particules choisies parmi les pigments, les nacres, les charges, et leurs mélanges.

**22.** Composition selon l'une quelconque des revendications précédentes, **caractérisée en ce qu'**elle comprend au moins une matière colorante.

**23.** Composition selon l'une quelconque des revendications précédentes, **caractérisée en ce qu'**elle se présente sous forme de bâton.

**24.** Utilisation cosmétique d'une composition cosmétique selon l'une quelconque des revendications précédentes, pour le soin et/ou le traitement de la peau, des lèvres et/ou des phanères, et en particulier pour l'hydratation des lèvres.

**25.** Utilisation cosmétique d'une composition cosmétique selon l'une quelconque des revendications 1 à 23, pour le maquillage de la peau, des lèvres et/ou des phanères.

**26.** Procédé cosmétique de traitement des lèvres gercées et/ou sèches, consistant à appliquer sur les lèvres, une composition cosmétique selon l'une quelconque des revendications 1 à 23.

Office européen des brevets

**RAPPORT PARTIEL DE RECHERCHE EUROPEENNE**
qui selon la règle 45 de la Convention sur le brevet européen est consideré, aux fins de la procédure ultérieure, comme le rapport de la recherche européenne

Numéro de la demande
EP 02 29 2479

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | WO 94 26234 A (IBAH, INC.) 24 novembre 1994 (1994-11-24) * revendication 1; exemple 1 * | 1,3,8, 10-14, 16,18, 19,23-26 | A61K7/02 |
| X | WO 99 59537 A (HENKEL KGAA) 25 novembre 1999 (1999-11-25) * revendication 1; exemples 28-35 * | 1 | |
| X | EP 0 980 684 A (BEIERSDORF AG) 23 février 2000 (2000-02-23) * revendication 1; exemples 6,11 * | 1 | |
| X | DE 196 43 063 A (HENKEL KGAA) 23 avril 1998 (1998-04-23) * le document en entier * | 1 | |
| X | DE 199 21 184 A (HENKEL KGAA) 9 novembre 2000 (2000-11-09) * le document en entier * | 1 | |

DOMAINES TECHNIQUES RECHERCHES (Int.Cl.7)

A61K
A61Q

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| LA HAYE | 7 février 2003 | Glikman, J-F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03 82 (P04C08)

Office européen des brevets

**RECHERCHE INCOMPLETE**
**FEUILLE SUPPLEMENTAIRE C**

Numéro de la demande
EP 02 29 2479

Revendications ayant fait
l'objet de recherches complètes:
1,3-26

Revendications n'ayant pas fait
l'objet de recherches:
2

Raison pour la limitation de la recherche:

La revendication 2 présente a trait à une composition définie (entre autres)
au moyen du paramètre suivant:
P1: une force de cisaillement

L'utilisation de ce paramètre est considérée , dans le présent contexte, comme menant à un manque de clarté au sens de l'Article 84 CBE. Il est impossible de comparer le paramètre que le déposant a choisi d'utiliser avec ce qui est révélé dans l'état de la technique. Le manque de clarté qui en découle est tel q'une recherche significative complète est impossible. Par conséquent, la recherche a été limitée aux sujets définis dans les autres revendications.

***********

## ANNEXE AU RAPPORT DE RECHERCHE EUROPEENNE RELATIF A LA DEMANDE DE BREVET EUROPEEN NO. EP 02 29 2479

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

07-02-2003

| Document brevet cité au rapport de recherche | | Date de publication | Membre(s) de la famille de brevet(s) | | | Date de publication |
|---|---|---|---|---|---|---|
| WO 9426234 | A | 24-11-1994 | AU | 6827794 | A | 12-12-1994 |
| | | | WO | 9426234 | A1 | 24-11-1994 |
| WO 9959537 | A | 25-11-1999 | DE | 19821691 | A1 | 18-11-1999 |
| | | | DE | 19832425 | A1 | 20-01-2000 |
| | | | AT | 221366 | T | 15-08-2002 |
| | | | AU | 3827499 | A | 06-12-1999 |
| | | | DE | 59902194 | D1 | 05-09-2002 |
| | | | WO | 9959537 | A1 | 25-11-1999 |
| | | | EP | 1076551 | A1 | 21-02-2001 |
| EP 980684 | A | 23-02-2000 | DE | 19837758 | A1 | 24-02-2000 |
| | | | EP | 0980684 | A2 | 23-02-2000 |
| DE 19643063 | A | 23-04-1998 | DE | 19643063 | A1 | 23-04-1998 |
| DE 19921184 | A | 09-11-2000 | DE | 19921184 | A1 | 09-11-2000 |
| | | | AU | 5210500 | A | 21-11-2000 |
| | | | WO | 0067703 | A2 | 16-11-2000 |
| | | | EP | 1183003 | A2 | 06-03-2002 |
| | | | JP | 2002544150 | T | 24-12-2002 |

EPO FORM P0460

Pour tout renseignement concernant cette annexe : voir Journal Officiel de l'Office européen des brevets, No.12/82